# EUROPEAN PATENT APPLICATION

(11) **EP 4 768 120 A1**
(43) Date of publication of application: **01.07.2026**
(21) Application number: 25773063.0
(22) Date of filing: 18.03.2025
(51) Int. Cl.: B01J 23/80, B01J 23/00, B01J 37/03, B01J 35/30, B01J 35/34, C07C 29/154, C07C 31/04

(54) **COPPER-BASED CATALYST, PREPARATION METHOD THEREFOR, AND USE THEREOF**

(30) Priority: 18.03.2024 CN 202410306520
(71) Applicant: ZHEJIANG INTELLIGENT TRANSPORTATION TECHNOLOGY INNOVATION CENTER, Zhejiang 315336 (CN); Zhejiang Geely Holding Group Co., Ltd., Hangzhou, Zhejiang 310000 (CN)
(72) Inventor: WANG, Xiaolong, Ningbo, Zhejiang 315336 (CN); LI, Yanpeng, Ningbo, Zhejiang 315336 (CN)
(74) Representative: Elzaburu S.L.P.
(86) International application number: PCT/CN2025/083161
(87) International publication number: WO 2025/195365

(57) **Abstract**

The present application discloses a copper-based catalyst, a method for preparing the same, and a use thereof. The method for preparing the copper-based catalyst includes: mixing zinc and aluminum precursors to obtain a first mixed solution, mixing copper and promoter precursors to obtain a second mixed solution, and preparing an alkaline precipitant solution, where the alkaline precipitant solution contains a silica precursor; mixing the first mixed solution with the alkaline precipitant solution, and precipitating zinc and aluminum components to obtain a first suspension; adding the second mixed solution and the alkaline precipitant solution to the first suspension, and precipitating copper component and a promoter to obtain a second suspension; and performing an aging treatment, a drying treatment, and a calcination treatment on the second suspension sequentially to obtain the copper-based catalyst.

## Description

### CROSS REFERENCE TO RELATED APPLICATIONS

The present application claims priority to Chinese Patent Application No. 202410306520.9, filed on March 18, 2024, the entire contents of which are incorporated herein by reference.

### TECHNICAL FIELD

The present application relates to the technical field of catalysts, and in particular to a copper-based catalyst, a method for preparing the same, and a use thereof.

### BACKGROUND

Methanol, as an important basic chemical raw material, can be used to synthesize various chemicals such as dimethyl ether, light olefins, aromatics, and acetic acid, and can also serve as an alternative fuel. China's methanol production mainly relies on the coal chemical route, which has a high carbon emission intensity and is detrimental to carbon reduction. In the context of "carbon peaking" and "carbon neutrality," developing methanol production through carbon dioxide hydrogenation is of great significance.

Copper-based catalysts are commonly used in the hydrogenation of carbon dioxide to methanol reaction, and these catalysts are often prepared by co-precipitation.

### SUMMARY

The main purpose of the present application is to provide a copper-based catalyst, a method for preparing the same, and a use thereof, aiming to solve the technical problems of poor stability and low catalytic hydrogenation activity of existing copper-based catalysts.

To achieve the above purpose, the present application provides a method for preparing a copper-based catalyst, including the following steps:
mixing a zinc precursor and an aluminum precursor to obtain a first mixed solution, mixing a copper precursor and a promoter precursor to obtain a second mixed solution, and preparing an alkaline precipitant solution, wherein the alkaline precipitant solution contains a silica precursor;
mixing the first mixed solution with the alkaline precipitant solution, and precipitating zinc component and aluminum component to obtain a first suspension;
adding the second mixed solution and the alkaline precipitant solution to the first suspension, and precipitating copper component and a promoter to obtain a second suspension; and
performing an aging treatment, a drying treatment and a calcination treatment on the second suspension in sequence to obtain the copper-based catalyst.

In an embodiment, a ratio of amount of aluminum to a sum of amounts of zinc, aluminum, copper and a promoter metal in the copper-based catalyst is 0.13 to 0.28.

In an embodiment, the copper-based catalyst includes copper oxide, aluminum oxide, zinc oxide, promoters, and silicon dioxide.

In an embodiment, a mass percentage of the promoter in the copper-based catalyst is 0.5% to 4%; and/or
a mass percentage of silicon dioxide in the copper-based catalyst is 0.5% to 4%.

In an embodiment, the promoter includes at least one of magnesium oxide, zirconium oxide, calcium oxide, strontium oxide and europium oxide.

In an embodiment, during precipitation of the zinc component and the aluminum component, a pH value of a reaction system is controlled to be 8 to 10; and/or
during precipitation of the copper component and the promoter, a pH value of a reaction system is controlled to be 8 to 10.

In an embodiment, the alkaline precipitant solution further includes sodium carbonate and sodium hydroxide, and a molar ratio of sodium carbonate to sodium hydroxide is 0.5 to 1.5.

In an embodiment, a temperature for the aging treatment is 50°C to 70°C, and a time for the aging treatment is 10h to 24h; and/or
a temperature for the calcination treatment is 300°C to 500°C, and a time for the aging treatment is 3h to 5h.

The present application also provides a copper-based catalyst prepared by the method for preparing the copper-based catalyst described above.

The present application also provides a copper-based catalyst prepared by the method for preparing the copper-based catalyst described above, or a use of the copper-based catalyst as described above in the hydrogenation of carbon dioxide to methanol.

The present application provides a copper-based catalyst, a method for preparing the same, and a use thereof. First, the co-precipitation process is carried out in two steps: first, zinc and aluminum are precipitated to form a zinc-aluminum hydrotalcite structure; then, copper and a promoter are precipitated. On the one hand, forming the zinc-aluminum hydrotalcite structure before precipitating copper and the promoter improves the dispersibility of the subsequently precipitated copper, resulting in more uniform copper particle size and thus suppressing the Oswald ripening effect. On the other hand, the zinc-aluminum hydrotalcite structure can provide a confinement effect, which suppresses the migration and sintering of copper components during subsequent catalytic processes. Furthermore, by inhibiting this migration, the growth of copper components during catalysis can be suppressed, thereby further inhibiting ripening and sintering. Second, the co-precipitation of copper and the promoter utilizes the hindering effect of the promoter to suppress copper migration, thereby inhibiting sintering caused by copper migration. Third, introducing a silica precursor into the alkaline precipitant solution allows for the in-situ introduction of hydrophobic silica during the co-precipitation process. On one hand, the introduction of silica improves the hydrophobicity of the copper-based catalyst, preventing accelerated catalyst deactivation due to the presence of water. On the other hand, compared to other silica introduction strategies, such as silane coupling agent modification or physical mixing, in-situ introduction of silica does not cover the active sites on the surface of the copper-based catalyst, resulting in a copper-based catalyst with higher catalytic hydrogenation activity. This overcomes the technical shortcomings of poor hydrothermal stability and low catalytic hydrogenation activity in copper-based catalysts. The present application, through an improved preparation method, enables the preparation of copper-based catalysts with higher hydrothermal stability and higher catalytic hydrogenation activity.

### BRIEF DESCRIPTION OF THE DRAWINGS

To more clearly illustrate the technical solutions in the embodiments of the present application or in the related art, accompanying drawings used in the description of the embodiments or the related art will be briefly introduced below. Obviously, the drawings described below are only some embodiments of the present application. For those skilled in the art, other drawings can be obtained based on the structures shown in these drawings without creative effort.

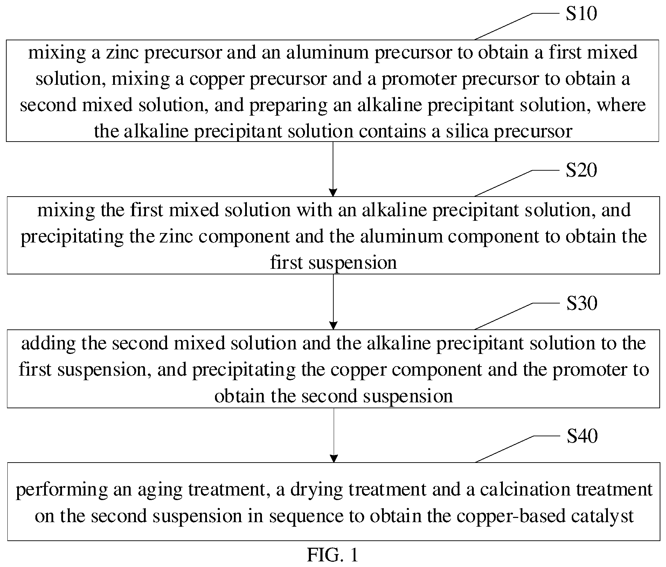

FIG. 1 is a schematic flowchart of a method for preparing a copper-based catalyst according to an embodiment of the present application.

The realization of the purpose, functional features and advantages of the present application will be further explained in conjunction with the embodiments and with reference to the accompanying drawings.

### DETAILED DESCRIPTION OF THE EMBODIMENTS

To make the above-mentioned objectives, features, and advantages of the present application more apparent and understandable, the technical solutions in the embodiments of the present application will be clearly and completely described below. Obviously, the described embodiments are merely some, not all, of the embodiments of the present application. Based on the embodiments in the present application, all other embodiments obtained by those skilled in the art without creative effort are within the scope of the present application.

However, copper-based catalysts prepared by co-precipitation generally have large copper particles with a wide particle size distribution. This uneven particle size easily leads to the Oswald ripening effect, resulting in catalyst sintering and deactivation. Furthermore, the carbon dioxide hydrogenation to methanol reaction occurs at high temperatures, while copper-based catalysts have poor hydrothermal stability. On the one hand, copper-based catalysts are prone to sintering at reaction temperatures above 200°C, leading to a reduction in active centers and catalyst deactivation. On the other hand, the generation of water during the carbon dioxide hydrogenation to methanol reaction accelerates copper sintering. The embodiment of the present application provides a method for preparing a copper-based catalyst. Referring to FIG. 1, the method for preparing the copper-based catalyst includes the following steps:
step S10, mixing a zinc precursor and an aluminum precursor to obtain a first mixed solution, mixing a copper precursor and a promoter precursor to obtain a second mixed solution, and preparing an alkaline precipitant solution, where the alkaline precipitant solution contains a silica precursor.

In this embodiment, the copper-based catalyst refers to a copper-zinc-aluminum system copper-based catalyst that can catalyze the hydrogenation of carbon dioxide to methanol. The copper-zinc-aluminum system copper-based catalyst includes at least copper oxide, aluminum oxide, and zinc oxide. Copper is the main active metal in the copper-based catalyst; zinc oxide can be used as a structural and electronic promoter, improving copper dispersion and increasing specific surface area; aluminum oxide can be used as a structural promoter, improving the specific surface area and mechanical stability of the catalyst. However, copper-based catalysts have poor stability and gradually deactivate during catalytic hydrogenation, mainly due to: deactivation caused by copper species migration; aging and sintering caused by uneven copper species particle size; and water-induced catalyst deactivation.

In an embodiment, the copper-based catalyst includes copper oxide, aluminum oxide, zinc oxide, a promoter, and silicon dioxide.

In this embodiment, the copper-based catalyst may include, in addition to copper oxide, aluminum oxide, and zinc oxide, a promoter and silica. The promoter is a substance that is inactive or has very low activity, but can alter some properties of the catalyst, such as electronic structure, ionic valence state, acidity/basicity, surface structure, and crystal size, thereby improving the activity, selectivity, anti-toxicity, or stability of the catalyst. The promoter can be at least one of a metal oxide or an inorganic non-metallic porous material. The silica is formed by reacting a silica precursor with a zinc precursor, an aluminum precursor, a copper precursor, and a promoter precursor, respectively. On one hand, silica is hydrophobic; therefore, its introduction can improve the hydrophobicity of the copper-based catalyst, preventing accelerated deactivation of the catalyst due to the presence of water. On the other hand, compared to other strategies for silica introduction, such as silane coupling agent modification or physical mixing, in-situ introduction of silica does not cover the active sites on the surface of the copper-based catalyst, resulting in a copper-based catalyst with higher catalytic hydrogenation activity.

In an embodiment, the promoter includes at least one of magnesium oxide, zirconium oxide, calcium oxide, strontium oxide, and europium oxide.

In this embodiment, the metal oxide exhibits excellent mechanical properties and good thermal conductivity, which not only improves the dispersibility of copper, inhibits copper migration, ensures uniform copper particle size, and avoids aging and sintering, but also enhances the mechanical stability and thermal conductivity of the copper-based catalyst, reducing hightemperature sintering. The hydrophobic nature of the metal oxide further prevents accelerated deactivation of the catalyst due to the presence of water, strengthens the interaction between copper and zinc, and thus improves catalytic hydrogenation activity. Therefore, the promoter is selected from at least one of magnesium oxide, zirconium oxide, calcium oxide, strontium oxide, and europium oxide.

The zinc precursor, used to prepare zinc oxide, can be a soluble salt of zinc, including at least one of zinc nitrate and zinc acetate. The aluminum precursor, used to prepare aluminum oxide, can be a soluble salt of aluminum, including at least one of aluminum nitrate and aluminum acetate. The copper precursor, used to prepare copper oxide, can be a soluble salt of copper, including at least one of copper nitrate and copper acetate. The promoter precursor, used to prepare the promoter, can be a soluble metal salt, including nitrates and acetates of metals such as magnesium, zirconium, calcium, strontium, and europium. The alkaline precipitant solution refers to a precipitant solution with an alkaline pH, and the alkaline precipitant solution contains a silica precursor. The silica precursor, used to prepare silica, can be an alkaline soluble silicate, including at least one of sodium silicate and potassium silicate.

In an embodiment, the alkaline precipitant solution further includes sodium carbonate and sodium hydroxide, where the molar ratio of sodium carbonate to sodium hydroxide is 0.5 to 1.5.

In this embodiment, by combining sodium carbonate and sodium hydroxide, a zinc-aluminum hydrotalcite structure can be formed when precipitating zinc and aluminum components. The molar ratio of sodium carbonate to sodium hydroxide is 0.5 to 1.5, for example, 0.5, 0.8, 1.0, 1.2, 1.5, etc.

As an example, step S10 includes: weighing zinc precursor, aluminum precursor, copper precursor, a promoter precursor, silica precursor, and precipitant in a certain proportion. The zinc and aluminum precursors are dissolved and mixed evenly to prepare a first mixed solution; the copper precursor and the promoter precursor are dissolved and mixed evenly to prepare a second mixed solution; and the silica precursor and precipitant are dissolved and mixed evenly to prepare a precipitant solution. It should be noted that the first mixed solution, the second mixed solution, and the precipitant solution are prepared independently, and the order of preparation is not limited. The amounts of zinc precursor, aluminum precursor, copper precursor, promoter precursor, silica precursor, and precipitant can be determined and adjusted in advance based on experimental test results or actual conditions, and this embodiment does not impose any restrictions on this.

In an embodiment, the ratio of the amount of aluminum to a sum of the amounts of zinc, aluminum, copper, and promoter metal in the copper-based catalyst is 0.13 to 0.28.

In this embodiment, the raw materials used to prepare the copper-based catalyst should ensure that the ratio of the amount of aluminum to the sum of the amounts of zinc, aluminum, copper, and promoter metal in the final copper-based catalyst is 0.13 to 0.28, for example, 0.13, 0.15, 0.2, 0.25, 0.28, etc. Under these conditions, the copper-based catalyst exhibits good stability and catalytic hydrogenation activity.

In an embodiment, the mass percentage of the promoter in the copper-based catalyst is 0.5% to 4%; and/or
the mass percentage of silicon dioxide in the copper-based catalyst is 0.5% to 4%.

In this embodiment, if the mass percentage of the promoter in the copper-based catalyst is too low, the improvement on catalyst performance is not significant. If the mass percentage of the promoter in the copper-based catalyst is too high, it may cover the active sites, which may lead to a decrease in catalytic hydrogenation activity. Therefore, the mass percentage of the promoter in the copper-based catalyst is determined to be 0.5% to 4%, such as 0.5%, 1%, 2%, 3%, 4%, etc.

If the mass percentage of silica in the copper-based catalyst is too low, the improvement in the hydrophobicity of the catalyst is not significant. If the mass percentage of silica in the copper-based catalyst is too high, it may cover the active sites, which may lead to a decrease in catalytic hydrogenation activity. Therefore, the mass percentage of silica in the copper-based catalyst is determined to be 0.5% to 4%, such as 0.5%, 1%, 2%, 3%, 4%, etc.

Step S20, mixing the first mixed solution with an alkaline precipitant solution, and precipitating the zinc component and the aluminum component to obtain the first suspension.

As an example, step S20 includes: taking a certain amount of pure water, and simultaneously and uniformly dripping the first mixed solution and the alkaline precipitant solution into the pure water so that the zinc component and the aluminum component co-precipitate to form a zinc-aluminum hydrotalcite structure. After the first mixed solution is completely added, a first suspension is obtained.

In an embodiment, during the precipitation of zinc and aluminum components, the pH value of the reaction system is controlled to be 8 to 10.

In this embodiment, the pH value of the reaction system can be tested simultaneously during the addition of the first mixed solution and the alkaline precipitant solution. By adjusting the dropping rate of the alkaline precipitant solution, the pH value of the reaction system can be controlled within the range of 8 to 10.

Step S30, adding the second mixed solution and the alkaline precipitant solution to the first suspension, and precipitating the copper component and the promoter to obtain the second suspension.

As an example, step S30 includes: after the addition of the first mixed solution is complete, adding the second mixed solution and the alkaline precipitant solution to the first suspension simultaneously and at a uniform rate, so that the copper component and the promoter co-precipitate, and the copper component and the promoter are dispersed and attached to the zinc-aluminum hydrotalcite. After the second mixed solution is added dropwise, a second suspension is obtained.

In an embodiment, during the precipitation of the copper component and the promoter, the pH value of the reaction system is controlled to be 8 to 10.

In this embodiment, the pH value of the reaction system can be tested simultaneously during the addition of the second mixed solution and the alkaline precipitant solution. By adjusting the dropping rate of the alkaline precipitant solution, the pH value of the reaction system can be controlled within the range of 8 to 10.

Step S40, performing an aging treatment, a drying treatment and a calcination treatment on the second suspension in sequence to obtain the copper-based catalyst.

As an example, step S40 includes: after the stepwise precipitation of zinc, aluminum, copper, and the promoter, sequentially performing the aging treatment on the second suspension; after the aging treatment, washing the precipitate; and drying the the washed precipitate and performing the calcination treatment to obtain the copper-based catalyst. The specific process parameters for the aging treatment, the drying treatment, and the calcination treatment can be determined and adjusted according to actual needs and test results, and is not limited in this embodiment.

In an embodiment, a temperature for the aging treatment is 50°C to 70°C, and a time for the aging treatment is 10 h to 24 h; and/or
the temperature for the calcination treatment is 300°C to 500°C, and the time for the aging treatment is 3h to 5h.

In this embodiment, the temperature for the aging treatment is 50°C to 70°C, such as 50°C, 60°C, 70°C, etc., and the time for the aging treatment is 10h to 24h, such as 10h, 15h, 20h, 24h, etc.

The temperature for the calcination treatment is 300°C to 500°C, for example 300°C, 350°C, 400°C, 450°C, 500°C, etc., and the time for the aging treatment is 3h to 5h, for example 3h, 4h, 5h, etc.

In this embodiment, the method for preparing the copper-based catalyst includes the following steps: mixing the zinc precursor and the aluminum precursor to obtain a first mixed solution, mixing the copper precursor and the promoter precursor to obtain a second mixed solution, and preparing an alkaline precipitant solution, where the alkaline precipitant solution contains a silica precursor; mixing the first mixed solution with the alkaline precipitant solution, and precipitating zinc component and aluminum component to obtain a first suspension; adding the second mixed solution and the alkaline precipitant solution to the first suspension, and precipitating the copper component and the promoter component to obtain a second suspension; and performing the aging treatment, the drying treatment, and the calcination treatment on the second suspension in sequence to obtain the copper-based catalyst. First, the co-precipitation process is divided into two steps. First, zinc and aluminum are precipitated to form a zinc-aluminum hydrotalcite structure. Then, copper and promoters are precipitated. On the one hand, forming the zinc-aluminum hydrotalcite structure before precipitating copper and promoters improves the dispersibility of the subsequently precipitated copper, resulting in more uniform copper particle size and thus suppressing the Oswald ripening effect. On the other hand, the zinc-aluminum hydrotalcite structure can provide a confinement effect, which inhibits the migration and sintering of the copper component during the subsequent catalytic process. Furthermore, by inhibiting copper migration, copper growth during catalysis can be suppressed, thereby also inhibiting sintering. Second, the co-precipitation of copper and promoters utilizes the hindering effect of the promoters to suppress copper migration, thereby inhibiting sintering caused by copper migration. Third, introducing a silica precursor into the alkaline precipitant solution allows for the in-situ introduction of hydrophobic silica during the co-precipitation process. On one hand, the introduction of silica improves the hydrophobicity of the copper-based catalyst, preventing accelerated catalyst deactivation due to the presence of water. On the other hand, compared to other silica introduction strategies, such as silane coupling agent modification or physical mixing, in-situ introduction of silica does not cover the active sites on the surface of the copper-based catalyst, resulting in a copper-based catalyst with higher catalytic hydrogenation activity. This overcomes the technical shortcomings of low hydrothermal stability and low catalytic hydrogenation activity in copper-based catalysts. The present application, through an improved preparation method, enables the preparation of copper-based catalysts with higher hydrothermal stability and higher catalytic hydrogenation activity.

The present application also provides a copper-based catalyst prepared by the method for preparing the copper-based catalyst described above.

The copper-based catalyst provided in the present application is prepared using the method described above, which solves the technical problems of poor stability and low catalytic hydrogenation activity of existing copper-based catalysts. Compared with the related art, the beneficial effects of the copper-based catalyst provided in the embodiment of the present application are the same as those of the method for preparing the copper-based catalyst provided in the above embodiments, and other technical features of this copper-based catalyst are the same as those disclosed in the methods of the above embodiments, and will not be repeated here.

The present application also provides a copper-based catalyst prepared by the method for preparing the same described above, or a use of the copper-based catalyst as described above in the hydrogenation of carbon dioxide to methanol.

In the present application, the use of the copper-based catalyst prepared by the method for preparing the same described above or the copper-based catalyst described above in the hydrogenation of carbon dioxide to methanol, solves the technical problems of poor stability and low catalytic hydrogenation activity of existing copper-based catalysts. Compared with the related art, the beneficial effects of the copper-based catalyst provided in the embodiment of the present application in the hydrogenation of carbon dioxide to methanol are the same as those of the method for preparing the copper-based catalyst provided in the above embodiments, and other technical features of the use of this copper-based catalyst in the hydrogenation of carbon dioxide to methanol are the same as those disclosed in the methods of the above embodiments, and will not be repeated here.

The present application is described in detail below with reference to specific embodiments and comparative examples. The following description is merely exemplary and not intended to limit the specific scope of the present application.

### Example 1

A copper-based catalyst is prepared using a two-step co-precipitation method. The mass ratio of Cu: ZnO: Al₂O₃ in the copper -based catalyst is 4: 3: 2. In the copper-based catalyst, ZrO₂ promoter and SiO₂ account for 1 wt.% and 0.5 wt.% of the total catalyst mass, respectively. The specific preparation method is as follows:
weighing 9.16 g of zinc nitrate hexahydrate and 12.88 g of aluminum nitrate nonahydrate, add 223 mL of deionized water, stirring and dissolving in a 500 mL beaker to obtain the first mixed solution; weighing 13.30 g of copper nitrate trihydrate and 0.38 g of zirconium nitrate and dissolving in 188 mL of deionized water to obtain the second mixed solution; and
weighing 26.5 g of anhydrous sodium carbonate, 10 g of sodium hydroxide and 0.27 g of sodium silicate, adding 500 ml of deionized water, stirring to dissolve, and forming a 1 mol/L alkaline precipitant solution.

First, the first mixed solution and the alkaline precipitant solution are simultaneously added dropwise to a three-necked flask containing 100 ml of deionized water. The suspension was kept at room temperature, maintaining a pH of 8. After the first mixed solution is completely added, the second mixed solution and the alkaline precipitant solution are added dropwise to the suspension, maintaining a pH of 8. After the addition is complete, the suspension is aged at 60°C for 16 h. The aged slurry is washed, dried, and then calcined in a muffle furnace at 300°C for 4 h to obtain the sample Cat1 catalyst.

### Example 2

A copper-based catalyst is prepared using a two-step co-precipitation method. The mass ratio of Cu: ZnO: Al₂O₃ in the copper -based catalyst is 4: 3: 2. In the copper-based catalyst, MgO promoter and SiO₂ account for 1 wt.% and 1 wt.% of the total catalyst mass, respectively. The specific preparation method is as follows:
weighing 9.11 g of zinc nitrate hexahydrate and 12.81 g of aluminum nitrate nonahydrate, adding 223 mL of deionized water, stirring and dissolving in a 500 mL beaker to obtain the first mixed solution; weighing 13.23 g of copper nitrate trihydrate and 0.51 g of magnesium nitrate and dissolving in 188 mL of deionized water to obtain the second mixed solution; and
weighing 29.44 g of anhydrous sodium carbonate, 8.8 g of sodium hydroxide and 0.54 g of sodium silicate, adding 500 ml of deionized water, stirring to dissolve, and forming a 1 mol/L alkaline precipitant solution.

First, the first mixed solution and the alkaline precipitant solution are simultaneously added dropwise to a three-necked flask containing 100 ml of deionized water. The suspension is kept at room temperature, maintaining a pH of 9. After the first mixed solution is completely added, the second mixed solution and the alkaline precipitant solution are added dropwise to the suspension, maintaining a pH of 9. After the addition is complete, the suspension is aged at 50°C for 24 h. The aged slurry is washed, dried, and then calcined in a muffle furnace at 500°C for 3 h to obtain the sample Cat2 catalyst.

### Example 3

A copper-based catalyst is prepared using a two-step co-precipitation method. The mass ratio of Cu: ZnO: Al₂O₃ in the copper-based catalyst is 4: 3: 2. In the copper-based catalyst, MgO promoter and SiO₂ account for 2 wt.% and 4 wt.% of the total catalyst mass, respectively. The specific preparation method is as follows:
weighing 8.74 g of zinc nitrate hexahydrate and 12.28 g of aluminum nitrate nonahydrate, adding 213 mL of deionized water, stirring and dissolving in a 500 mL beaker to obtain the first mixed solution; weighing 12.69 g of copper nitrate trihydrate and 1.02 g of magnesium nitrate and dissolving in 187 mL of deionized water to obtain the second mixed solution; and
weighing 29.44 g of anhydrous sodium carbonate, 8.8 g of sodium hydroxide and 2.16 g of sodium silicate, adding 500 ml of deionized water, stirring to dissolve, and forming a 1 mol/L alkaline precipitant solution.

First, the first mixed solution and the alkaline precipitant solution are simultaneously added dropwise to a three-necked flask containing 100 ml of deionized water. The solution is kept at room temperature, maintaining the pH of the suspension at 10. After the first mixed solution was completely added, the second mixed solution and the alkaline precipitant solution were added dropwise to the suspension, maintaining the pH of the suspension at 10. After the addition is complete, the suspension is aged at 70°C for 12 h. The aged slurry is washed, dried, and then calcined in a muffle furnace at 500°C for 4 h to obtain the sample Cat3 catalyst.

### Example 4

A copper-based catalyst is prepared using a two-step co-precipitation method. The mass ratio of Cu: ZnO: Al₂O₃ in the copper -based catalyst is 5: 3: 2. In the copper-based catalyst, MgO promoter and SiO₂ account for 1 wt.% and 4 wt.% of the total catalyst mass, respectively. The specific preparation method is as follows:
weighing 7.95 g of zinc nitrate hexahydrate and 11.18 g of aluminum nitrate nonahydrate, adding 194 mL of deionized water, stirring and dissolving in a 500 mL beaker to obtain the first mixed solution; weighing 14.43 g of copper nitrate trihydrate and 0.51 g of magnesium nitrate and dissolving in 205 mL of deionized water to obtain the second mixed solution; and
weighing 31.8 g of anhydrous sodium carbonate, 8 g of sodium hydroxide and 2.16 g of sodium silicate, adding 500 ml of deionized water, stirring to dissolve, and forming a 1 mol/L alkaline precipitant solution.

First, the first mixed solution and the alkaline precipitant solution are simultaneously added dropwise to a three-necked flask containing 100 ml of deionized water. The suspension was kept at room temperature, maintaining a pH of 9.5. After the first mixed solution is completely added, the second mixed solution and the alkaline precipitant solution are added dropwise to the suspension, maintaining the pH at 9.5. After the addition is complete, the suspension is aged at 70°C for 12 h. The aged slurry is then washed, dried, and calcined in a muffle furnace at 500°C for 4 h to obtain the sample Cat4 catalyst.

### Example 5

A copper-based catalyst is prepared using a two-step co-precipitation method. The mass ratio of Cu: ZnO: Al₂O₃ in the copper -based catalyst is 5: 3: 2. In the copper-based catalyst, SrO₂ promoter and SiO₂ account for 1 wt.% and 2 wt.% of the total catalyst mass, respectively. The specific preparation method is as follows:
weighing 8.12 g of zinc nitrate hexahydrate and 11.41 g of aluminum nitrate nonahydrate, adding 164 mL of deionized water, stirring and dissolving in a 500 mL beaker to obtain the first mixed solution; weighing 14.74 g of copper nitrate trihydrate and 0.16 g of strontium nitrate and dissolving in 229 mL of deionized water to obtain the second mixed solution; and
weighing 31.8 g of anhydrous sodium carbonate, 8 g of sodium hydroxide and 1.08 g of sodium silicate, adding 500 ml of deionized water, stirring to dissolve, and forming a 1 mol/L alkaline precipitant solution.

First, the first mixed solution and the alkaline precipitant solution are simultaneously added dropwise to a three-necked flask containing 100 ml of deionized water. The suspension was kept at room temperature, maintaining a pH of 8. After the first mixed solution is completely added, the second mixed solution and the alkaline precipitant solution are added dropwise to the suspension, maintaining a pH of 8. After the addition is complete, the suspension is aged at 60°C for 16 h. The aged slurry is washed, dried, and then calcined in a muffle furnace at 300°C for 4 h to obtain the sample Cat5 catalyst.

### Example 6

A copper-based catalyst is prepared using a two-step co-precipitation method. The mass ratio of Cu: ZnO: Al₂O₃ in the copper-based catalyst is 2: 1: 1. In the copper-based catalyst, MgO promoter and SiO₂ account for 1 wt.% and 5 wt.% of the total catalyst mass, respectively. The specific preparation method is as follows:
weighing 6.56 g of zinc nitrate hexahydrate and 13.82 g of aluminum nitrate nonahydrate, adding 201 mL of deionized water, stirring and dissolving in a 500 mL beaker to obtain the first mixed solution; weighing 14.28 g of copper nitrate trihydrate and 1.02 g of magnesium nitrate and dissolving in 209 mL of deionized water to obtain the second mixed solution; and
weighing 22.71 g of anhydrous sodium carbonate, 11.43 g of sodium hydroxide and 1.08 g of sodium silicate, adding 500 ml of deionized water, stirring to dissolve, and forming a 1 mol/L alkaline precipitant solution.

First, the first mixed solution and the alkaline precipitant solution are simultaneously added dropwise to a three-necked flask containing 100 ml of deionized water. The suspension is kept at room temperature, maintaining a pH of 8. After the first mixed solution is completely added, the second mixed solution and the alkaline precipitant solution are added dropwise to the suspension, maintaining a pH of 8. After the addition is complete, the suspension is aged at 60°C for 20 h. The aged slurry is washed, dried, and then calcined in a muffle furnace at 300°C for 4 h to obtain the sample Cat6 catalyst.

### Example 7

A copper-based catalyst is prepared using a two-step co-precipitation method. The mass ratio of Cu: ZnO: Al₂O₃ in the copper -based catalyst is 5: 3: 1. In the copper-based catalyst, MgO promoter and SiO₂ account for 1 wt.% and 1 wt.% of the total catalyst mass, respectively. The specific preparation method is as follows:
weighing 9.11 g of zinc nitrate hexahydrate and 6.41 g of aluminum nitrate nonahydrate, adding 165 mL of deionized water, stirring and dissolving in a 500 mL beaker to obtain the first mixed solution; weighing 16.54 g of copper nitrate trihydrate and 0.51 g of magnesium nitrate and dissolving in 234 mL of deionized water to obtain the second mixed solution; and
weighing 29.44 g of anhydrous sodium carbonate, 8.8 g of sodium hydroxide and 0.54 g of sodium silicate, adding 500 ml of deionized water, stirring to dissolve, and forming a 1 mol/L alkaline precipitant solution.

First, the first mixed solution and the alkaline precipitant solution are simultaneously added dropwise to a three-necked flask containing 100 ml of deionized water. The suspension is kept at room temperature, maintaining a pH of 8. After the first mixed solution is completely added, the second mixed solution and the alkaline precipitant solution are added dropwise to the suspension, maintaining a pH of 8. After the addition is complete, the suspension is aged at 60°C for 20 h. The aged slurry is washed, dried, and then calcined in a muffle furnace at 300°C for 4 h to obtain the sample Cat7 catalyst.

### Example 8

A copper-based catalyst is prepared using a two-step co-precipitation method. The mass ratio of Cu: ZnO: Al₂O₃ in the copper -based catalyst is 5: 3: 1. In the copper-based catalyst, CaO promoter and SiO₂ account for 0.5 wt.% and 3 wt.% of the total catalyst mass, respectively. The specific preparation method is as follows:
weighing 8.97 g of zinc nitrate hexahydrate and 6.31 g of aluminum nitrate nonahydrate, adding 163 mL of deionized water, stirring and dissolving in a 500 mL beaker to obtain the first mixed solution; weighing 16.28 g of copper nitrate trihydrate and 0.07 g of calcium nitrate and dissolving in 224 mL of deionized water to obtain the second mixed solution; and
weighing 22.71 g of anhydrous sodium carbonate, 11.43 g of sodium hydroxide and 0.54 g of sodium silicate, adding 500 ml of deionized water, stirring to dissolve, and forming a 1 mol/L alkaline precipitant solution.

First, the first mixed solution and the alkaline precipitant solution are simultaneously added dropwise to a three-necked flask containing 100 ml of deionized water. The solution is kept at room temperature, maintaining the pH of 9. After the first mixed solution is completely added, the second mixed solution and the alkaline precipitant solution are added dropwise to the suspension, maintaining the pH of 9. After the addition is complete, the suspension is aged at 60°C for 20 h. The aged slurry is washed, dried, and then calcined in a muffle furnace at 400°C for 4 h to obtain the sample Cat8 catalyst.

### Example 9

A copper-based catalyst is prepared using a two-step co-precipitation method. The mass ratio of Cu: ZnO: Al₂O₃ in the copper -based catalyst is 5: 3: 1. In the copper-based catalyst, ZrO₂ promoter and SiO₂ account for 1 wt.% and 2 wt.% of the total catalyst mass, respectively. The specific preparation method is as follows:
weighing 9.02 g of zinc nitrate hexahydrate and 6.34 g of aluminum nitrate nonahydrate, adding 164 mL of deionized water, stirring and dissolving in a 500 mL beaker to obtain the first mixed solution; weighing 16.37 g of copper nitrate trihydrate and 0.38 g of zirconium nitrate and dissolving in 188 mL of deionized water to obtain the second mixed solution; and
weighing 26.5 g of anhydrous sodium carbonate, 10 g of sodium hydroxide and 0.11 g of sodium silicate, adding 400 ml of deionized water, stirring to dissolve, and forming a 1 mol/L alkaline precipitant solution.

First, the first mixed solution and the alkaline precipitant solution are simultaneously added dropwise to a three-necked flask containing 100 ml of deionized water. The suspension is kept at room temperature, maintaining a pH of 8. After the first mixed solution is completely added, the second mixed solution and the alkaline precipitant solution are added dropwise to the suspension, maintaining a pH of 8. After the addition is complete, the suspension is aged at 60°C for 16 h. The aged slurry is washed, dried, and then calcined in a muffle furnace at 300°C for 5 h to obtain the sample Cat9 catalyst.

### Comparative Example 1

A copper-based catalyst is prepared using a two-step co-precipitation method. The mass ratio of Cu: ZnO: Al₂O₃ in the copper-based catalyst is 4: 3: 2, without the addition of any promoters or SiO₂. The specific preparation method is as follows :
weighing 9.3 g of zinc nitrate hexahydrate and 13.07 g of aluminum nitrate nonahydrate, adding 227 mL of deionized water, stirring and dissolving in a 500 mL beaker to obtain the first mixed solution; weighing 13.50 g of copper nitrate trihydrate and dissolving in 185 mL of deionized water to obtain the second mixed solution; and
weighing 26.5 g of anhydrous sodium carbonate and 10 g of sodium hydroxide, adding 500 ml of deionized water, stirring to dissolve, and forming a 1 mol/L alkaline precipitant solution.

First, the first mixed solution and the alkaline precipitant solution are simultaneously added dropwise to a three-necked flask containing 100 ml of deionized water. The suspension is kept at room temperature, maintaining a pH of 9. After the first mixed solution is completely added, the second mixed solution and the alkaline precipitant solution are added dropwise to the suspension, maintaining a pH of 9. After the addition is complete, the suspension is aged at 60°C for 16 h. The aged slurry is washed, dried, and then calcined in a muffle furnace at 400°C for 4 h to obtain the sample Cat10 catalyst.

### Comparative Example 2

A copper-based catalyst is prepared using a two-step co-precipitation method. The mass ratio of Cu: ZnO: Al₂O₃ in the copper-based catalyst is 5: 3: 1. MgO is added as a promoter at 1 wt.% of the total catalyst mass, but SiO₂ is not added. The specific preparation method is as follows:
weighing 9.21 g of zinc nitrate hexahydrate and 6.47 g of aluminum nitrate nonahydrate, adding 167 mL of deionized water, stirring and dissolving in a 500 mL beaker to obtain the first mixed solution; weighing 16.71 g of copper nitrate trihydrate and 0.51 g of magnesium nitrate and dissolving in 236 mL of deionized water to obtain the second mixed solution;
weighing 26.5 g of anhydrous sodium carbonate and 10 g of sodium hydroxide, adding 500 ml of deionized water, stirring to dissolve, and forming a 1 mol/L alkaline precipitant solution.

First, the first mixed solution and the alkaline precipitant solution are simultaneously added dropwise to a three-necked flask containing 100 ml of deionized water. The suspension is kept at room temperature, maintaining a pH of 8. After the first mixed solution is completely added, the second mixed solution and the alkaline precipitant solution are added dropwise to the suspension, maintaining a pH of 8. After the addition is complete, the suspension is aged at 60°C for 16 h. The aged slurry is washed, dried, and then calcined in a muffle furnace at 400°C for 4 h to obtain the sample Cat11 catalyst.

### Comparative Example 3

A copper-based catalyst is prepared using a two-step co-precipitation method. The mass ratio of Cu: ZnO: Al₂O₃ in the copper-based catalyst was 5: 3: 2. 1 wt.% SiO₂ of the total catalyst mass is added, and no promoters are added. The specific preparation method is as follows:
weighing 8.2 g of zinc nitrate hexahydrate and 11.53 g of aluminum nitrate nonahydrate, adding 200 mL of deionized water, stirring and dissolving in a 500 mL beaker to obtain the first mixed solution; weighing 14.88 g of copper nitrate trihydrate and dissolving in 205 mL of deionized water to obtain the second mixed solution; and
weighing 26.5 g of anhydrous sodium carbonate, 10 g of sodium hydroxide and 1.08 g of sodium silicate, adding 500 ml of deionized water, stirring to dissolve, and forming a 1 mol/L alkaline precipitant solution.

First, the first mixed solution and the alkaline precipitant solution are simultaneously added dropwise to a three-necked flask containing 100 ml of deionized water. The suspension is kept at room temperature, maintaining a pH of 9. After the first mixed solution is completely added, the second mixed solution and the alkaline precipitant solution are added dropwise to the suspension, maintaining a pH of 9. After the addition is complete, the suspension is aged at 50°C for 20 h. The aged slurry is washed, dried, and then calcined in a muffle furnace at 500°C for 4 h to obtain the sample Cat12 catalyst.

### Comparative Example 4

A copper-based catalyst is prepared using a one-step co-precipitation method. The mass ratio of Cu: ZnO: Al₂O₃ in the copper-based catalyst is 4: 3: 2. In the copper-based catalyst, ZrO₂ promoter and SiO₂ account for 1 wt.% and 0.5 wt.% of the total catalyst mass, respectively. The specific preparation method is as follows:
weighing 9.16 g zinc nitrate hexahydrate, 12.88 g aluminum nitrate nonahydrate, 13.30 g copper nitrate trihydrate, and 0.38 g zirconium nitrate, and dissolving them in 411 mL of deionized water to obtain a mixed solution; and
weigh 26.5 g of anhydrous sodium carbonate, 10 g of sodium hydroxide and 0.27 g of sodium silicate, adding 500 ml of deionized water, stirring to dissolve, and forming a 1 mol/L alkaline precipitant solution.

The mixed solution and alkaline precipitant solution are simultaneously added dropwise to a three-necked flask containing 100 ml of deionized water. The suspension is kept at room temperature, maintaining a pH of 8. After the addition is completed, the suspension is aged at 60°C for 16 h. The aged slurry is washed, dried, and then calcined in a muffle furnace at 300°C for 4 h to obtain the sample Cat13 catalyst.

The above-described embodiments and comparative catalysts are applied to the carbon dioxide hydrogenation to methanol reaction, and their reaction performance is tested. The reaction performance test conditions are: a reaction temperature of 250°C, a reaction pressure of 5MPa, reaction space velocity of 10000 mL·h-1·gcat-1, and H₂ to CO₂ flow ratio of 3: 1.

The test results are shown in Table 1.

**[Table 1_sm_0001]**

| Sample Name | CO₂ conversion rate (%) | Methanol selectivity (%) | Methanol space-time yield (g _{MeOH} ·g _{cat} ⁻¹ ·h ⁻¹) | Deactivation rate (%/h) |
|---|---|---|---|---|
| Cat.1 | 24.1 | 56.5 | 0.486 | 0.083 |
| Cat.2 | 21.4 | 53 | 0.405 | 0.078 |
| Cat.3 | 20.6 | 51.9 | 0.382 | 0.046 |
| Cat.4 | 22.1 | 56.4 | 0.445 | 0.045 |
| Cat.5 | 23.6 | 59.1 | 0.498 | 0.072 |
| Cat.6 | 21.9 | 54.3 | 0.425 | 0.062 |
| Cat.7 | 24.6 | 62.1 | 0.546 | 0.059 |
| Cat.8 | 22.5 | 53 | 0.426 | 0.089 |
| Cat.9 | 24.1 | 60.2 | 0.518 | 0.075 |
| Cat.10 | 18.2 | 48.5 | 0.315 | 0.129 |
| Cat.11 | 23.9 | 58.8 | 0.502 | 0.081 |
| Cat.12 | 21.6 | 52.8 | 0.407 | 0.076 |
| Cat.13 | 20.5 | 50.8 | 0.372 | 0.091 |

Referring to Table 1, a comparison of Cat. 10 and Cat. 1-9 shows that promoters and silica can effectively improve the CO₂ conversion rate, methanol selectivity, and methanol space-time yield of copper-based catalysts, and effectively slow down the deactivation rate of copper-based catalysts. A comparison of Cat. 11 and Cat. 1-9 shows that silica can effectively improve the CO₂ conversion rate, methanol selectivity, and methanol space-time yield of copper-based catalysts, and effectively slow down the deactivation rate of copper-based catalysts. A comparison of Cat. 12 and Cat. 1-9 shows that promoters can effectively improve the CO₂ conversion, methanol selectivity, and methanol space-time yield of copper-based catalysts, and effectively slow down the deactivation rate of copper-based catalysts. A comparison of Cat. 13 and Cat. 1-9 shows that stepwise precipitation can effectively improve the CO₂ conversion, methanol selectivity, and methanol space-time yield of copper-based catalysts, and effectively slow down the deactivation rate of copper-based catalysts.

The above are merely embodiments of the present application and do not limit the scope of the present application. Any equivalent structural or procedural transformations made using the description of the present application, or direct or indirect applications in other related technical fields, are similarly included within the scope of the present application.

## Claims

1. A method for preparing a copper-based catalyst, **characterized by** comprising:
mixing a zinc precursor and an aluminum precursor to obtain a first mixed solution, mixing a copper precursor and a promoter precursor to obtain a second mixed solution, and preparing an alkaline precipitant solution, wherein the alkaline precipitant solution contains a silica precursor;
mixing the first mixed solution with the alkaline precipitant solution, and precipitating zinc component and aluminum component to obtain a first suspension;
adding the second mixed solution and the alkaline precipitant solution to the first suspension, and precipitating copper component and a promoter to obtain a second suspension; and
performing an aging treatment, a drying treatment and a calcination treatment on the second suspension in sequence to obtain the copper-based catalyst.

2. The method for preparing the copper-based catalyst according to claim 1, wherein a ratio of amount of aluminum to a sum of amounts of zinc, aluminum, copper and a promoter metal in the copper-based catalyst is 0.13 to 0.28.

3. The method for preparing the copper-based catalyst according to claim 1, wherein the copper-based catalyst comprises copper oxide, aluminum oxide, zinc oxide, promoters, and silicon dioxide.

4. The method for preparing the copper-based catalyst according to claim 3, wherein a mass percentage of the promoter in the copper-based catalyst is 0.5% to 4%; and/or
a mass percentage of silicon dioxide in the copper-based catalyst is 0.5% to 4%.

5. The method for preparing the copper-based catalyst according to claim 3, wherein the promoter comprises at least one of magnesium oxide, zirconium oxide, calcium oxide, strontium oxide and europium oxide.

6. The method for preparing the copper-based catalyst according to claim 1, wherein during precipitation of the zinc component and the aluminum component, a pH value of a reaction system is controlled to be 8 to 10; and/or
during precipitation of the copper component and the promoter, a pH value of a reaction system is controlled to be 8 to 10.

7. The method for preparing the copper-based catalyst according to claim 1, wherein the alkaline precipitant solution further comprises sodium carbonate and sodium hydroxide, and a molar ratio of sodium carbonate to sodium hydroxide is 0.5 to 1.5.

8. The method for preparing the copper-based catalyst according to any one of claims 1 to 7, wherein a temperature for the aging treatment is 50°C to 70°C, and a time for the aging treatment is 10h to 24h; and/or
a temperature for the calcination treatment is 300°C to 500°C, and a time for the aging treatment is 3h to 5h.

9. A copper-based catalyst, **characterized in that** the copper-based catalyst is prepared by the method for preparing the copper-based catalyst according to any one of claims 1 to 8.

10. A use of the copper-based catalyst prepared by the method for preparing the copper-based catalyst according to any one of claims 1 to 8 or the copper-based catalyst according to claim 9 in hydrogenation of carbon dioxide to methanol.
